# EUROPEAN PATENT APPLICATION

(11) **EP 2 599 480 A1**
(43) Date of publication of application: **05.06.2013**
(21) Application number: 11009595.7
(22) Date of filing: 03.12.2011
(51) Int. Cl.: A61K 31/185, A61K 31/194, A61K 31/255, A61K 31/433, A61K 31/655, A61P 31/12, A61P 31/16

(54) **Compounds for the treatment of influenza**

(71) Applicant: Universität Innsbruck, 6020 Innsbruck (AT)
(72) Inventor: Kirchmair, Johannes, Dr., Cambridge CB1 3EL (GB); Schmidtke, Michaela, Dr., 07743 Jena (DE); Liedl, Klaus, 6020 Innsbruck (AT); Rollinger, Judith, M., Dr., 6020 Innsbruck (AT)
(74) Representative: Schrell, Andreas

(57) **Abstract**

The present invention relates to compounds and pharmaceutical compositions comprising said compounds for use in the treatment or prevention of an influenza virus infection. In particular, the disclosed compounds inhibit viral neuraminidase, preferably of influenza viruses resistant to known neuraminidase inhibitors.

## Description

The present invention relates to compounds and pharmaceutical compositions comprising said compounds for use in the treatment or prevention of an influenza virus infection. In particular, the disclosed compounds inhibit viral neuraminidase, preferably of influenza viruses resistant to known neuraminidase inhibitors.

Seasonal influenza, also known as flu, is one of the most severe infectious diseases, causing an annual world-wide death toll of an estimated 250 000 to 500 000 people that can be incommensurably higher in pandemic years. The young, elderly and also patients suffering from chronic diseases are particularly threatened by the associated risk of bacterial pneumonia.

Influenza viruses A and B belong to the family of Orthomyxoviridae and are enveloped viruses with a single-stranded, negative-sense RNA comprising eight segments, each encoding one or two viral proteins. Of particular interest are two of those proteins, namely hemagglutinin (abbreviated in the following as HA or H) and neuraminidase (abbreviated in the following as NA or N) which are viral surface membrane glycoproteins. The observed different combinations of those two viral proteins are used to name subtypes of the viruses (e. g. H5N1), with 16 known different HA types (H1 to H16) and nine different NA types (N1 to N9) known to date for the influenza A serotype. During the infectious cycle HA is involved in the attachment of viral particles to host cell surface glycoproteins, whereas NA plays a role in the release of the newly synthesised viral particles from the host cell. In particular, NA, a membrane-bound homotetrameric enzyme, releases the mature viral particles by cleaving terminal sialic acid residues on the host cell surface proteoglycans, bound by HA, thus inhibiting viral aggregation. Hence, the pathogenicity of influenza A viruses depends on the function of influenza NA.

Viruses of the influenza A serotype are the most virulent human pathogens, having caused several severe influenza pandemics, for example H1N1 (1918), H2N2 (1957) and H3N2 (1968), as a result of their high transmission rates. Also, H5N1, known for causing bird flu, shows high virulence and lethality. Its lack of efficient human-to-human transmission has limited its impact on public health so far. However, the pronounced potential of H5N1 for viral re-assortment and mutation has raised major concerns about public health. More recently, the 2009 pandemic H1N1 influenza A virus (H1N1v) immerged by re-assortment of avian, human and classical swine viruses as well as European swine viruses. Fortunately, the pandemic of this highly transmissive new influenza type turned out to be less dramatic than initially anticipated due to its low virulence.

Since influenza NA is the enzyme facilitating the detachment of the mature virus from the host cell and its active site is relatively well conserved, it was established as the primary target for drug treatment of influenza using structure-based antiviral drug development. This led to the characterisation of inhibitors such as zanamivir (Relenza®) and oseltamivir (Tamiflu®), which both are approved influenza drugs, the latter being the first orally bioavailable NA inhibitor. Both of these drugs competitively inhibit the NAs sialidase function as sialic acid analogues, thus stalling the viral infectious cycle. Due to antigenic drift and/or mutation an increased incidence of influenza viruses resistant to such NA inhibitors (NAls) has been reported since the flu season of 2007/2008. In particular, H1N1 viruses resistant to oseltamivir carrying a H274Y mutation have been observed. In 2009, however, they were mostly replaced by the influenza A virus H1N1v which is NA inhibitor-susceptible. Still, the spontaneous and unpredictable development of drug resistance points and the deficiency of a broad portfolio of anti-influenza drugs available for the prevention or treatment of future pandemics pose a major risk. Thus, there is a huge demand for the development of new anti-influenza drugs able to efficiently target influenza viruses in general and in particular those that are resistant to known drugs.

The first crystal structure of a group-1 NA (N1) was revealed in 2006 showing the existence of a "150-loop" that, under various conditions, closes upon ligand-binding to the active site through movement of the 150-loop. This drew particular attention to such extended conformational shifts of the 150- but also the 245- and 430-loops that allow for great flexibility of the tetrameric viral enzyme. Thus, by using experimental studies on protein structure as well as theoretical studies new "attack points for drug design", such as the opening of the binding pocket due to the flexibility of the 150-, 245- and 430-loops of the NA molecule were identified.

Using representative conformations of the N1 binding site (including the flexible loop regions), extracted from a clustering analysis of the N1 molecular dynamics simulations for computational solvent mapping the binding affinity of small, solvent-sized probe molecules was assessed previously revealing the presence of novel druggable hot spots in those loop regions. With a related strategy employing an ensemble-based approach to virtual screening using a set of eight N1 receptor structures consisting of two crystal structures and the three most dominant clusters extracted from the molecular dynamics simulations of the explicitly solvated tetrameric N1 system with or without oseltamivir bound to screen the National Cancer Institute (NCI) diversity set with AutoDock 4 and the refinement technique RCS (Relaxed Complex Scheme), several substances capable of inhibiting N1 were characterised. These compounds are disclosed in WO 2009/128964. Although capable of inhibiting N1, the compounds disclosed in WO 2009/128964 are predicted to be mostly not more potent than known NA inhibitors like oseltamivir and zanamivir.

Thus, the technical problem underlying the present invention is to provide compounds having an improved activity in the treatment or prevention of a viral infection, in particular an influenza infection, more particular an influenza virus A or B infection. In the context of the present invention an improved activity is meant to encompass higher efficiency in terms of an inhibitory action against the virus or in terms of showing an inhibitory action against resistant viruses or both. Thus, the technical problem underlying the present invention is in particular to provide further compounds capable of inhibiting influenza NA, in particular of influenza viruses resistant to known NA inhibitors.

This technical problem is solved by the subject matter of the independent claims. Accordingly, the present invention relates in particular to a compound having a formula selected from the group consisting of: 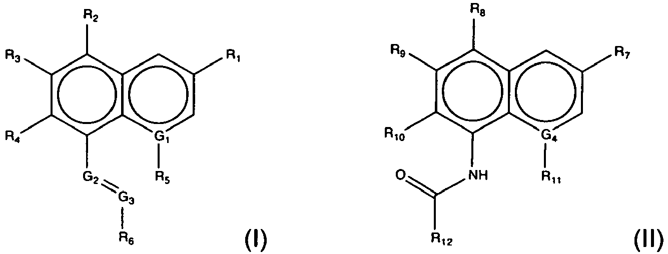 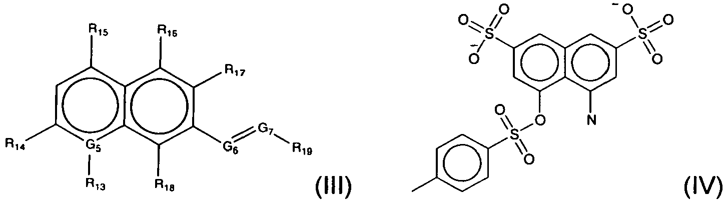 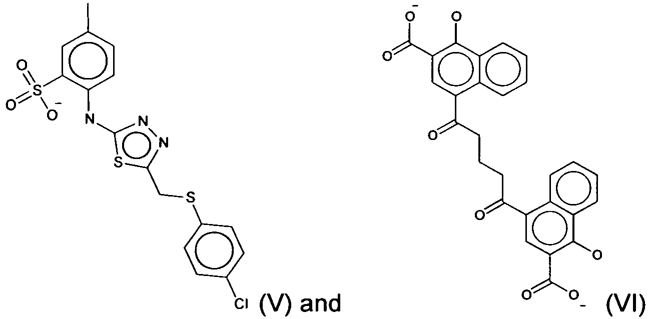 for use in the treatment or prevention of a viral infection, wherein in (I):
**G1, G2** and **G3** are each independently selected from the group consisting of N and C, wherein in case **G1** is N **G1** is not substituted; preferably **G1** is C or substituted C;
**R1**, **R2**, **R3** and **R5** are each independently selected from the group consisting of -SO₃H, -COOH, -OH, -NH₂ and -H;
**R4** is selected from the group consisting of -OH, -NH₂ and -H;
wherein at least one of **R1**, **R2**, **R3** and **R5** is -COOH or -SO₃H or **R4** is -OH or -NH₂; and
**R6** is selected from the group consisting of C1-C6 alkyl chain, phenyl, substituted phenyl, 5-membered or 6-membered heterocycles, substituted 5-membered or 6-membered heterocycles, substituted or non-substituted 5,5 fused bicycles, substituted or non-substituted 6,5 fused bicycles, substituted or non-substituted 6,4 fused bicycles and substituted or non-substituted naphthalenes linked in position 2 or heterocyclic derivatives thereof linked in the corresponding position;
and wherein in (**II**):
**G4** is selected from the group consisting of N and C, wherein in case **G4** is N **G4** is not substituted; preferably **G4** is C or substituted C;
**R7**, **R8**, **R9** and **R11** are each independently selected from the group consisting of -SO₃H, -COOH, -OH, -NH₂ and -H, wherein preferably at least one of **R7**, **R8**, **R9** and **R11** is -COOH or -SO₃H;
**R10** is selected from the group consisting of -OH, -NH₂ and -H; and
**R12** is selected from the group consisting of C1-C6 alkyl chain, phenyl, substituted phenyl, 5-membered or 6-membered heterocycles and substituted 5-membered or 6-membered heterocycles;
and wherein in (**III**):
**G5**, **G6** and **G7** are each independently selected from the group consisting of N and C, wherein in case **G5** is N **G5** is not substituted; preferably **G5** is C or substituted C;
**R13**, **R14** and **R16** are each independently selected from the group consisting of -SO₃H, -COOH and -H,
**R17** is selected from the group consisting of -SO₃H, -COOH, -OH and -H;
wherein preferably at least one of **R13**, **R14**, **R16** and **R17** is -COOH or -SO₃H;
**R15** and **R18** are each independently selected from the group consisting of -OH, -NH₂ and -H; and
**R19** is selected from the group consisting of C1-C6 alkyl chain, phenyl, substituted phenyl, 5-membered or 6-membered heterocycles, substituted 5-membered or 6-membered heterocycles, substituted or non-substituted 5,5 fused bicycles, substituted or non-substituted 6,5 fused bicycles, substituted or non-substituted 6,4 fused bicycles and substituted or non-substituted naphthalenes linked in position 2 or heterocyclic derivatives thereof linked in the corresponding position;
or a pharmaceutically acceptable salt, prodrug or tautomer thereof.

Surprisingly, the compounds of the present invention exhibit significant and, in a preferred embodiment, resistance-breaking inhibitory activity on influenza NA. This was investigated by using in vitro assays of distinct NA strains to elucidate the bioactivity profile of the present compounds, in particular with regard toward oseltamivir-resistant strains. This study revealed that all of the tested compounds of the present invention exhibit an IC₅₀ value of ≤ 9 µM for the influenza virus strain A/PR/8/34, more importantly, most of them even having an IC₅₀ value of < 0.5 µM for this strain. The individual IC₅₀ values are given in table 1 below in µM. IC₅₀ meaning the concentration of inhibitor causing a 50 % inhibition of viral NA activity.

In one embodiment of the present invention, the present compounds share a naphthalene carboxylic/sulfonic acid terminus, connected to a central planar system via a diazenyl linker. However, several distinctive structural features are observable. The linker, e.g., may be exchanged by related structural elements and also by more flexible chains. Also the connectivity among corresponding moieties may vary significantly. Without wishing to be bound by theory, the observed very potent inhibition caused by the present compounds might inter alia be due to the moieties located at positions **R6, R12** and **R19** of (**I**), (**II**) and (**III**), respectively, as well as the corresponding residues of (**IV**), (**V**) and (**VI**), which mostly are substituted phenyl or, rather representing exceptions, substituted naphthalenes linked in position 2. All of these residues are relatively small and slender. Since these moieties come to lie in the middle of the respective compounds this likely coincides with a narrowing or constriction of the binding pocket. Thus, compounds exhibiting rather broad moieties in these positions, for instance naphthalene rests linked in position 1, might not fit properly into the binding pocket in this area.

In the context of the present invention, the term "naphthalenes linked in position 2" is understood to mean that the C atom at position 2 or β of a non-substituted naphthalene, which is not a C atom directly adjacent to the C atoms shared by the two rings, is linked to the base molecule represented by the generic formulas (I) or (III). The term "corresponding position" in heterocyclic derivatives of naphthalenes is understood to mean that the numbering of positions is not changed due to the heteroatom in the heterocyclic derivative. Thus, the position in a heterocyclic derivative corresponding to position 2 in a naphthalene is the same position as in the naphthalene irrespective of the heteroatom. Similarly, the numbering of positions is not changed due to any substituents of the naphthalene or heterocyclic derivative.

In a preferred embodiment, the present invention relates to a compound having the formula 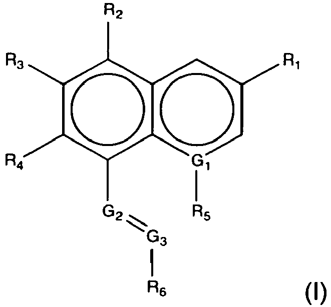 for use in the treatment or prevention of a viral infection, wherein:
**G1**, **G2** and **G3** are each independently selected from the group consisting of N and C, wherein in case **G1** is N **G1** is not substituted; preferably **G1** is C or substituted C;
**R1, R2, R3** and **R5** are each independently selected from the group consisting of -SO₃H, -COOH, -OH, -NH₂ and -H;
**R4** is selected from the group consisting of -OH, -NH₂ and -H;
wherein at least one of **R1, R2, R3** and **R5** is -COOH or -SO₃H or **R4** is -OH or -NH₂; and
**R6** is selected from the group consisting of C1-C6 alkyl chain, phenyl, substituted phenyl, 5-membered or 6-membered heterocycles, substituted 5-membered or 6-membered heterocycles, substituted or non-substituted 5,5 fused bicycles, substituted or non-substituted 6,5 fused bicycles, substituted or non-substituted 6,4 fused bicycles and substituted or non-substituted naphthalenes linked in position 2 or heterocyclic derivatives thereof linked in the corresponding position;
or a pharmaceutically acceptable salt, prodrug or tautomer thereof.

In a further preferred embodiment -COOH is preferred over -SO₃H for **R1**, **R2**, **R3** and **R5**, and preferably less than three acidic moieties are present in total at **R1**, **R2**, **R3** and **R5**. **R4** is preferably -OH. Particularly preferred are -SO₃H or -COOH at **R1** and/or **R3**. Further preferred is a combination of **R3** being -COOH and **R4** being -OH. **R6** is preferably selected from the group consisting of p-nitrophenyl, m-nitrophenyl, p-aminophenyl, and p,N,N-dialkyl-aminophenyl, in particular in combination with **R1** being -SO₃H, **R2**, **R3** and **R5** being -H and **R4** being -OH.

In a particularly preferred embodiment the present invention relates to a compound having the formula 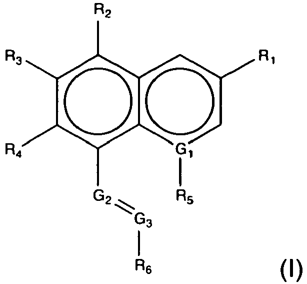 for use in the treatment or prevention of a viral infection, wherein:
**G1** is C;
**G2** is N;
**R1** is selected from the group consisting of -SO₃H and -H;
**R2** is -H;
**R3** is selected from the group consisting of -H, -COOH and -SO₃H;
**R4** is selected from the group consisting of -H and -OH;
**R5** is selected from the group consisting of -H, -OH and -SO₃H;
wherein at least one of **R1**, **R3** and **R5** is -COOH or -SO₃H; and
**R6** is selected from the group consisting of 4-phenyldiazenylphenyl, 4-(N-(6-methylpyridin-2-yl)sulfamoyl)phenyl, 4-(N-(2,6-dimethylpyrimidin-4-yl)sulfamoyl)phenyl, 4-sulfamoylphenyl, 4-sulfamoylpyridazine, 4-(N-(5-methyl-1,3,4-thiadiazol-2-yl)sulfamoyl)phenyl, 4-(N-(pyridin-2-yl)sulfamoyl)phenyl, 4-(N-(3,4-dimethylisoxazolidin-5-yl)sulfamoyl)phenyl, 3,p-dimethylphenyl, 3-nitrophenyl, 4-nitrophenyl, 4-N,N-dimethylphenyl, 2-methyl-4-((2-methyl-4-sulfophenyl)diazenyl)phenyl, 4-(N-(pyrimidin-2-yl)sulfamoyl)phenyl, 4-(N-carbamimidoylsulfamoyl)phenyl, 4-(N-(thiazol-2-yl)sulfamoyl)phenyl, 4-sulfophenyl and 8-amino-1-hydroxy-3,6-disulfonaphthalen-2-yl;.
or a pharmaceutically acceptable salt, prodrug or tautomer thereof.

In a further particularly preferred embodiment the present invention relates to a compound for use in the treatment or prevention of a viral infection selected from the group consisting of the NCI compounds 65545, 134133, 134140, 134137, 134131, 134134, 134132, 134141, 45541, 37200, 66221, 134136, 134139, 134135, 45539, 45538, 45540, 134138, 162530 and 37199, or a pharmaceutically acceptable salt, prodrug or tautomer thereof. Particularly preferred are the compounds NCI 65545 and NCI 37200, or a pharmaceutically acceptable salt, prodrug or tautomer thereof.

In the context of the present invention NCI compounds are compounds listed in the NCI database (National Cancer Institute) as of 24^{th} of August 2009. Formulas of the preferred NCI compounds are listed in Table 1, below.

In a preferred embodiment the present invention relates to a compound having the formula 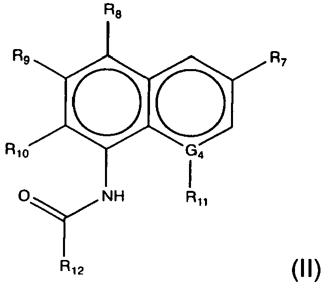 for use in the treatment or prevention of a viral infection, wherein:
**G4** is selected from the group consisting of N and C, wherein in case **G4** is N **G4** is not substituted; preferably **G4** is C or substituted C.
**R7**, **R8**, **R9** and **R11** are each independently selected from the group consisting of -SO₃H, -COOH, -OH, -NH₂ and -H, wherein preferably at least one of **R7**, **R8**, **R9** and **R11** is -COOH or -SO₃H;
**R10** is selected from the group consisting of -OH, -NH₂ and -H; and
**R12** is selected from the group consisting of C1-C6 alkyl chain, phenyl, substituted phenyl, 5-membered or 6-membered heterocycles and substituted 5-membered or 6-membered heterocycles;
or a pharmaceutically acceptable salt, prodrug or tautomer thereof.

In a further preferred embodiment -COOH is preferred over -SO₃H for **R7**, **R8** and **R9** and preferably less than three acidic moieties are present in total at **R7**, **R8** and **R9**. **R10** is preferably -OH. Particularly preferred are -SO₃H or -COOH at **R7** and/or **R9**.

In a particularly preferred embodiment the present invention relates to a compound having the formula 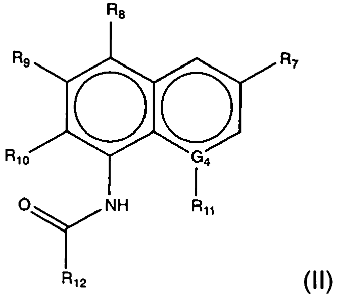 for use in the treatment or prevention of a viral infection, wherein:
**G4** is C;
**R7**, **R8** and **R9** are each independently selected from the group consisting of -SO₃H and -H;
**R10** is -H;
**R11** is selected from the group consisting of -OH and -H; and
**R12** is selected from the group consisting of pentyl, 4-aminophenyl and phenyl;
or a pharmaceutically acceptable salt, prodrug or tautomer thereof.

In a further particularly preferred embodiment the present invention relates to a compound for use in the treatment or prevention of a viral infection selected from the group consisting of the NCI compounds 97290, 37180, 37176, 37609, 16169, 37172 and 74489, or a pharmaceutically acceptable salt, prodrug or tautomer thereof.

In a preferred embodiment the present invention relates to a compound having the formula 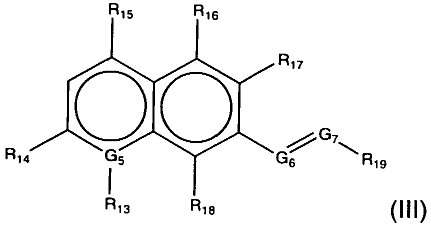 for use in the treatment or prevention of a viral infection, wherein:
**G5**, **G6** and **G7** are each independently selected from the group consisting of N and C, wherein in case **G5** is N **G5** is not substituted; preferably **G5** is C or substituted C;
**R13**, **R14** and **R16** are each independently selected from the group consisting of -SO₃H, -COOH and -H,
**R17** is selected from the group consisting of -SO₃H, -COOH, -OH and -H;
wherein preferably at least one of **R13**, **R14**, **R16** and **R17** is -COOH or -SO₃H;
**R15** and **R18** are each independently selected from the group consisting of -OH, -NH₂ and -H; and
**R19** is selected from the group consisting of C1-C6 alkyl chain, phenyl, substituted phenyl, 5-membered or 6-membered heterocycles, substituted 5-membered or 6-membered heterocycles, substituted or non-substituted 5,5 fused bicycles, substituted or non-substituted 6,5 fused bicycles, substituted or non-substituted 6,4 fused bicycles and substituted or non-substituted naphthalenes linked in position 2 or heterocyclic derivatives thereof linked in the corresponding position;
or a pharmaceutically acceptable salt, prodrug or tautomer thereof.

In a further preferred embodiment -COOH is preferred for **R13**, **R14**, **R16** and **R17** and preferably less than three acidic moieties are present in total at **R13**, **R14**, **R15** and **R16**. -OH is preferred for **R15** and **R18** is preferably -NH₂. Particularly preferred are -SO₃H or -COOH at **R14** or **R17**, in particular -COOH.

In a particularly preferred embodiment the present invention relates to a compound having the formula 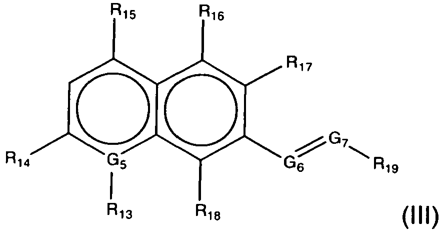 for use in the treatment or prevention of a viral infection, wherein:
**G6** is N;
**R13** is selected from the group consisting of -H and SO₃H;
**R14** is selected from the group consisting of -H and SO₃H;
**R15** is selected from the group consisting of -H and -OH;
**R17** is selected from the group consisting of -H, -OH and SO₃H; and
**R19** is selected from the group consisting of 6-sulfonaphthalen-2-yl, 4-(N-(5-methyl-1,3,4-thiadiazol-2-yl)sulfamoyl)phenyl, 4-(N-(4-methylpyrimidin-2-yl)sulfamoyl)phenyl, 4-(N-(pyridin-2-yl)sulfamoyl)phenyl, 4-(N-(thiazol-2-yl)sulfamoyl)phenyl, phenyl and 4-((4-ethoxyphenyl)diazenyl)phenyl;
or a pharmaceutically acceptable salt, prodrug or tautomer thereof.

In a further particularly preferred embodiment the present invention relates to a compound for use in the treatment or prevention of a viral infection selected from the group consisting of the NCI compounds 87877, 134142, 134144, 134145, 31348, 45549, 30456 and 37220, or a pharmaceutically acceptable salt, prodrug or tautomer thereof.

In a further particularly preferred embodiment the compound for use in the treatment or prevention of a viral infection is 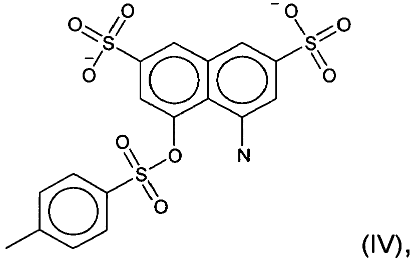 NCI compound 16168,
or a pharmaceutically acceptable salt, prodrug or tautomer thereof.

In a further particularly preferred embodiment the compound for use in the treatment or prevention of a viral infection is 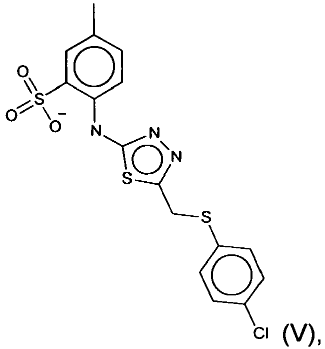 NCl compound 369693,
or a pharmaceutically acceptable salt, prodrug or tautomer thereof.

In a further particularly preferred embodiment the compound for use in the treatment or prevention of a viral infection is 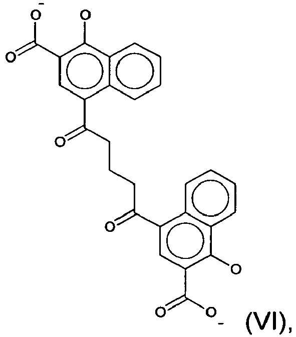 NCl compound 292216,
or a pharmaceutically acceptable salt, prodrug or tautomer thereof.

In the context of the present invention the viral infection is preferably an influenza infection. Most preferably, the influenza virus infection is caused by influenza virus A or B, preferably an influenza A virus.

In a preferred embodiment, the influenza virus infection is caused by an influenza A virus with a group-1 NA (N) selected from the group consisting of N1, N4, N5 and N8. That means, the compounds according to the present invention are preferably capable of inhibiting a group-1 NA, including N1, N4, N5 and N8, regardless of the HA subtype the NA is combined with in the influenza virus. Particularly preferred, the compounds of the present invention are capable to inhibit the NA N1. Thus, in a particularly preferred embodiment the influenza virus infection is caused by a H1N1 virus or a H5N1 virus. Advantageously, the compounds according to the present invention can preferably also be used in the treatment or prevention of an influenza infection, wherein the influenza virus causing the infection, in particular its NA, is resistant to known NA inhibitors, in particular oseltamivir.

Thus, the compounds according to the present invention are in a preferred embodiment for use in the treatment or prevention of a viral infection, in particular an influenza infection by inhibiting the NA (N) of a virus, in particular an influenza virus. That means, the influenza infection is treated or prevented by the compounds of the present invention preferably by inhibiting the NA of a virus, preferably an influenza virus.

The technical problem underlying the present invention is also solved by pharmaceutically acceptable salts, prodrugs or tautomers of the compounds according to the present invention.

In the context of the present invention a pharmaceutically acceptable salt is an ionic compound composed of cations and anions, wherein at least the cation or anion is the ionised form of a compound of the present invention and wherein said salt has at least the pharmacological and biological activity of the compounds of the present invention. In the context of the present invention the term "salt" also encompasses the ion, i. e. the anion or cation, alone as long as said ion is the cationic or anionic form of a compound of the present invention.

In the context of the present invention a prodrug is an inactive or at least less active form of a compound of the present invention administered as a pharmacological substance which is then metabolised in vivo into the active metabolite, preferably the compound of the present invention. Thus, in the context of the present invention a prodrug of the present invention is a substance which, after having been metabolised in vivo, is capable of exhibiting the same pharmacological and biological activity as the compound of the present invention.

In the context of the present invention a tautomer is an isomer of a compound of the present invention that is capable of readily interconverting into the compounds of the present invention by a tautomerisation reaction. Thus, the present invention also relates to tautomers of the present compounds which exhibit at least the biological and pharmacological activity as the compounds of the present invention.

In the context of the present invention the terms "pharmacological and biological activity" or "pharmacological and biological function" refer to the capability of the compounds of the present invention to prevent and/or treat a viral infection, in particular an influenza infection, more particularly a viral infection caused by an influenza virus A or B, preferably an influenza virus A. In the context of the present invention a pharmacological and biological activity is preferably determined by a chemiluminescence-based NA inhibition assay, preferably as described in example 2. Preferably a compound is considered to have a biological and pharmacological activity such as the compounds of the present invention, if it exhibits in the chemiluminescence-based NA inhibition assay as described in example 2 an IC₅₀ value of < 10 µM, preferably of < 5 µM, more preferably of < 2, most preferably of < 1 µM.

The terms "treatment" or "prevention" as used herein, refer to eliciting a desired pharmacological and/or physiological effect in a patient, preferably human or animal patient. These effects are prophylactic or preventive in terms of completely or partially preventing a disease and/or its symptoms, and therapeutic in terms of partially or completely curing a disease and/or to alleviate or ameliorate symptoms of a disease.

The present invention further relates to a pharmaceutical composition comprising at least one compound according to the present invention and a pharmaceutically acceptable carrier, also called excipient. The term "carrier" or "excipient" as used herein is understood to mean any substance, not itself a therapeutic agent, used as a carrier, diluent, adjuvant, or vehicle for delivery of a therapeutic compound to a subject or added to a pharmaceutical composition to improve its handling or storage properties or to permit formation of a dose unit of the composition into a discrete product such as a capsule or tablet suitable for oral administration. Thus, such excipients are used to facilitate processing of the compounds into preparations which can be used pharmaceutically. Acceptable excipients are well known to the skilled person.

In general, excipients can include diluents, disintegrants, binding agents, adhesives, wetting agents, polymers, lubricants, glidants, substances added to mask a disagreeable taste or odour, flavours, dyes, fragrances, surface active agents, film forming substances, and substances added to improve appearance of the composition, for instance smoothing agents. In particular, such excipients can include lactose, sucrose, starch powder, maize starch or derivatives thereof, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinyl-pyrrolidone, and/or polyvinyl alcohol, saline, dextrose, mannitol, lactose, lecithin, albumin, sodium glutamate, cysteine hydrochloride, and the like. Examples of suitable excipients for soft gelatin capsules include vegetable oils, waxes, fats, semi-solid and liquid polyols. Coconut oil, olive oil, sesame oil, peanut oil or soya can also be used as suspension agents or lubricants. Additionally or alternatively, cellulose acetate phthalate as a derivative of a carbohydrate such as cellulose, or methylacetate-methacrylate copolymer as a derivative of polyvinyl, or plasticizers such as ester phthalates and the like may be used as suspension agents. Suitable excipients for the preparation of solutions and syrups include, for example, water, polyols, sucrose, invert sugar and glucose. Suitable excipients for injectable solutions include, for instance, water, alcohols, polyols, glycerol, and vegetable oils. Sterile compositions for injection can be formulated according to known pharmaceutical practice. For example, dissolution or suspension of the present compound in a vehicle such as water or naturally occurring vegetable oil like sesame, peanut, or cottonseed oil or a synthetic fatty vehicle like ethyl oleate or the like may be desired. Further, buffers, preservatives, antioxidants and the like can be incorporated according to accepted pharmaceutical practice.

If the manufacture of pharmaceutical compositions according to the present invention involves intimate mixing of the pharmaceutical excipients and the present compound in its pharmaceutically acceptable salt form, it may be desirable to use pharmaceutical excipients which are non-basic, that is, either acidic or neutral excipients.

The pharmaceutical compositions can additionally include preservatives, solubilizers, stabilizers, wetting agents, emulsifiers, sweeteners, colorants, flavourings, buffers, coating agents, or antioxidants. For example, sodium benzoate, ascorbic acid and esters of p-hydroxy benzoic acid may be used as preservatives. The compounds of the present invention can also be made in microencapsulated form according to known processes.

For hydrophobic compounds of the present invention, a preferred pharmaceutical excipient may be a co-solvent system comprising benzyl alcohol, a non-polar surfactant, a water-miscible organic polymer, and an aqueous phase. A common co-solvent system used is the VPD co-solvent system, which is a solution of 3% w/v benzyl alcohol, 8% w/v of the non-polar surfactant Polysorbate 80™, and 65% w/v polyethylene glycol 300, made up to volume in absolute ethanol. Of course, the proportions of a co-solvent system may be varied considerably without changing its solubility and toxicity characteristics. Furthermore, other low-toxicity non-polar surfactants may be used instead of Polysorbate 80™. Also, the fraction size of polyethylene glycol may be varied or other biocompatible polymers may replace polyethylene glycol, e.g. polyvinyl pyrrolidone, whereas other sugars or polysaccharides may substitute for dextrose. Alternatively, other delivery systems for hydrophobic pharmaceutical compounds may be employed. Liposomes and emulsions are well known examples of delivery vehicles for hydrophobic drugs. Organic solvents such as dimethylsulfoxide may also be employed, although usually at the cost of greater toxicity.

Furthermore, the compounds of the present invention may be delivered using a sustained-release system, such as semipermeable matrices of solid hydrophobic polymers containing them. Various sustained-release materials have been established and are well known. Sustained-release capsules may, depending on their chemical nature, release the present compounds for at least a few weeks. Alternatively, the compounds may also be formulated as a depot preparation. Such long acting formulations may be delivered by implantation, for example subcutaneously or intramuscularly, or by intramuscular injection. Thus the present compounds may be formulated with suitable polymeric or hydrophobic materials, for example as an emulsion in an acceptable oil, or ion exchange resins, or as sparingly soluble derivatives, for example, as a sparingly soluble salt.

The pharmaceutical compositions according to the present invention can further include any other agents that provide improved transfer, delivery or tolerance. Such agents can include powders, pastes, ointments, jellies, waxes, oils, lipids, DNA conjugates, anhydrous absorption pastes, oil-in-water and water-in-oil emulsions, emulsions carbowax (polyethylene glycols of various molecular weights), semi-solid gels, and semi-solid mixtures containing carbowax.

Any of the foregoing pharmaceutical formulations may be used in accordance with the present invention, provided that the at least one compound according to the present invention in the formulation is not inactivated by the formulation and the formulation is physiologically compatible and tolerable with the route of administration.

The pharmaceutical compositions of the present invention may be manufactured in a manner that is known per se, e.g., by means of conventional mixing, dissolving, granulating, dragee-making, levigating, emulsifying, encapsulating, entrapping or tabletting processes.

Proper formulation of the pharmaceutical compositions according to the present invention is dependent upon the route of administration chosen. Any of the well-known techniques, carriers, and excipients may be used as suitable and as understood in the art. Preferably, the pharmaceutical composition is formulated so as to allow the present compounds contained therein to be bioavailable upon administration of the composition to a patient.

The present compounds may be formulated for parenteral administration by injection, e.g., by bolus injection or continuous infusion by conventional methods, either as liquid solutions or suspensions, solid forms suitable for solution or suspension in liquid prior to injection, or as emulsions. Suitable excipients are, for example, water, saline, dextrose, mannitol, lactose, lecithin, albumin, sodium glutamate, cysteine hydrochloride. In addition, the injectable pharmaceutical compositions may contain minor amounts of nontoxic auxiliary substances, such as wetting agents, pH buffering agents, and the like. Physiologically compatible buffers include, for example, Hanks's solution, Ringer's solution, or physiological saline buffer. Also, absorption enhancing preparations, for example, liposomes, may be utilized. Formulations for injection may be presented in unit dosage form, e.g., in ampoules or in multi-dose containers, with an added preservative. Thus, pharmaceutical compositions for parenteral administration include aqueous solutions of the present compounds in water-soluble form. Aqueous injection suspensions may contain substances which increase the viscosity of the suspension, such as sodium carboxymethyl-cellulose, sorbitol, or dextran. Optionally, the suspension may also contain suitable stabilizers or agents which increase the solubility of the compounds to allow for the preparation of highly concentrated solutions. Alternatively, suspensions of the present compounds may be prepared as oily injection suspensions. Suitable lipophilic solvents include oils such as sesame oil, soybean oil, grapefruit or almond oils, or synthetic fatty acid esters, such as ethyl oleate or triglycerides, or liposomes. Thus, the pharmaceutical compositions may take such forms as suspensions, solutions or emulsions in oily or aqueous vehicles, and may contain formulatory agents such as suspending, stabilizing and/or dispersing agents. Alternatively, the present compounds may be in powder form for reconstitution with a suitable vehicle, e.g., sterile pyrogen-free water, before use.

For transmucosal administration, appropriate penetrants able to penetrate the targeted barrier may be used in the formulation.

For oral administration, the present compounds can be formulated by combining them with pharmaceutically acceptable excipients, e. g. as described above. Such carriers enable the present compounds of the invention to be formulated as tablets, pills, dragees, capsules, liquids, gels, syrups, slurries, suspensions and the like, for oral ingestion by a patient. Thus, pharmaceutical compositions for oral use can be obtained by combining the present compounds with solid excipients, optionally grinding a resulting mixture, and processing the mixture of granules, after optionally adding further suitable auxiliaries, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethyl-cellulose, and/or polyvinylpyrrolidone (PVP). Optionally, disintegrating agents may be added, such as the cross-linked polyvinyl pyrrolidone, agar, or alginic acid or a salt thereof such as sodium alginate. Dragee cores, of course, can be provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different doses of the present compounds.

Pharmaceutical compositions which can be used orally further include push-fit capsules made of gelatin, as well as soft, sealed capsules made of gelatin and a plasticizer, such as glycerol or sorbitol. The push-fit capsules can contain the present compounds in admixture with a filler such as lactose, binders such as starches, and/or lubricants such as talc or magnesium stearate and, optionally, stabilizers. In soft capsules, the present compounds may be dissolved or suspended in suitable liquids, such as oils, liquid paraffin, or liquid polyethylene glycols.

For buccal administration, the pharmaceutical compositions may take the form of tablets or lozenges formulated in conventional manner.

For administration by inhalation, the compounds according to the present invention are conveniently delivered in the form of an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. If a pressurized aerosol is used the dosage unit may be determined by providing a valve to deliver a metered amount. Capsules and cartridges of, e.g., gelatin for use in an inhaler or insufflator may be preferably formulated containing a powder mix of the present compound and a suitable powder base such as lactose or starch.

Further suitable administration ways include intraocular, intranasal, and intraauricular delivery. Suitable penetrants for these uses are generally known in the art. Pharmaceutical compositions for intraocular delivery include aqueous ophthalmic solutions of the compounds in water-soluble form, such as eyedrops, or in gellan gum; ophthalmic ointments; ophthalmic suspensions, such as microparticulates, compound-containing small polymeric particles that are suspended in a liquid carrier medium; and ocular inserts. Such suitable pharmaceutical formulations are preferably formulated to be sterile, isotonic and buffered for stability. Pharmaceutical compositions for intranasal delivery may also include drops and sprays often prepared to simulate nasal secretions to ensure maintenance of normal ciliary action. As well-known to those skilled in the art, suitable formulations are preferably isotonic, slightly buffered to maintain a pH of 5.0 to 7.0, preferably 5.5 to 6.5, and include antimicrobial preservatives and appropriate stabilizers. Pharmaceutical formulations for intraauricular delivery include suspensions and ointments for topical application in the ear. Common solvents for such aural formulations include glycerin and water.

The compounds of the present invention may also be formulated for rectal delivery, for example as suppositories or retention enemas, therefore, e.g., containing conventional suppository bases such as cocoa butter or other glycerides.

If the compounds of the present invention are intended to be administered intracellular they may be administered using techniques well known. For example, the compounds may be encapsulated into liposomes. All molecules present in an aqueous solution at the time of liposome formation are incorporated into the aqueous interior. The liposomal contents are both protected from the external microenvironment and, because liposomes fuse with cell membranes, are efficiently delivered into the cell cytoplasm. The liposome may be coated with a tissue-specific antibody. The liposomes will be targeted to and taken up selectively by the desired organ.

Additionally, the pharmaceutical compositions of the present invention may be combined with other compositions that contain other therapeutic or diagnostic agents.

In one embodiment, the compounds of the present invention can be administered to a patient, preferably a human patient, alone or in pharmaceutical compositions where they are mixed with other active ingredients, such as in combination therapy. Thus, the compounds according to the present invention are preferably used individually, in combination with each other, that means two or more of the compounds according to the present invention, or in combination with other pharmaceutically, biologically or therapeutically active substances, for the preparation of a pharmaceutical composition for the treatment or prevention of a viral infection, in particular an influenza virus infection in mammals, most preferably humans.

Suitable methods of administration include, for example, (a) administration though oral pathways, including administration in a capsule, tablet, granule, spray, syrup, or other such forms; (b) administration through non-oral pathways such as rectal, vaginal, intraurethral, intraocular, intranasal, or intraauricular, including administration as an aqueous suspension, an oily preparation or the like or as a drip, spray, suppository, salve or ointment; (c) parenteral administration, preferably via injection, for example, subcutaneously, intraperitoneally, intravenously, intramuscularly, intradermally, intraorbitally, intracapsularly, intraspinally, intrasternally, intramedullary, intrathecal, intraocular, including infusion pump delivery; (d) local administration such as by injection directly in the renal or cardiac area, e.g., by depot implantation; as well as (e) topical administration, e. g. transdermal, including electrotransport, patches, for prolonged and/or timed, pulsed administration at a predetermined rate; all as deemed appropriate by those of skill in the art for delivering the compounds of the invention to a patient.

Preferably, at least one compound according to the present invention is contained in the pharmaceutical composition in an amount effective to achieve its intended purpose which is the treatment or prevention of a viral infection, preferably an influenza infection. The therapeutically effective amount of the compounds required as a dose will depend on the route of administration, the type of animal, including human, being treated, and the physical characteristics of the specific animal under consideration. The dose can be tailored to achieve the desired effect and depends on factors such as weight, diet, concurrent medication and other factors. More specifically, a therapeutically effective amount as used herein means an amount of the compound effective to prevent, alleviate or ameliorate symptoms of disease, in particular a viral infection, or prolong the survival of the subject being treated. Determination of a therapeutically effective amount is well within the capability of those skilled in the art using routine pharmacological methods. For example, acceptable in vitro studies can be used to establish useful doses and routes of administration of the compositions of the present invention. Further, administration may be commenced at low dosage levels, with dosage levels being increased until the desired effect is achieved.

Thus, the dosage may range broadly, depending upon the desired effect, route of administration and so on. A suitable dosage of the compounds of the present invention may be from 0.01 µg/kg to 1 g/kg body weight, administered by a route considered suitable. A more preferred dosage is from 1 µg/kg to 500 mg/kg, including 10 µg/kg, 100 µg/kg, 1 mg/kg, 10 mg/kg, 20 mg/kg, 50 mg/kg, 100 mg/kg, 200 mg/kg, 300 mg/kg, 400 mg/kg, and various ranges within these amounts. The daily dosage regimen for an adult human patient may be, for example, an oral dose of from 0.1 mg to 2000 mg of the compound, preferably from 1 mg to 500 mg, more preferably from 5 to 200 mg. Further preferred, an oral composition may be administered in a dosage of up to 750 mg/kg, in particular up to 500 mg/kg, preferably up to 300 mg/kg, most preferred up to 150 mg/kg. In other embodiments, an intravenous, subcutaneous, or intramuscular dose of the present compounds may be from 0.01 mg to 100 mg, preferably from 0.1 mg to 60 mg, more preferably from 1 to 40 mg. In particular, an intravenous composition may be administered in a dosage of from 5 mg/kg to 25 mg/kg. In cases of administration of a pharmaceutically acceptable salt of the present compounds, dosages may be calculated as the free base. As will be understood by those of skill in the art, in certain situations it may be necessary to administer the compounds disclosed herein in amounts that exceed, or even far exceed, the above-stated, preferred dosage range in order to effectively treat particularly aggressive forms of the viral infection.

The dosage may be a single one or a series of two or more given in the course of one or more days, as is needed by the patient. In some embodiments, the pharmaceutical composition may be administered 1 to 4 times per day. Alternatively, the pharmaceutical compositions of the invention may be administered by continuous intravenous infusion, preferably at a dose of up to 1000 mg per day of the compound. In some embodiments, the compounds will be administered for a period of continuous therapy, for example for a week or more, or for months.

Dosage amount and interval may also be adjusted in order to provide plasma levels of the active moiety which are sufficient to maintain the modulating effects, or minimal effective concentration (MEC). The MEC will vary for each compound but can be estimated from in vitro data. Dosages necessary to achieve the MEC depend on individual characteristics and route of administration. Dosage intervals can also be determined using MEC value. Pharmaceutical compositions should be administered using a regimen which maintains plasma levels above the MEC for 10 to 90% of the time, preferably between 30 to 90% and most preferably between 50 to 90%.

Compounds of the present invention can be evaluated for efficacy and toxicity using known methods. For example, the toxicology of a particular compound, may be established by determining in vitro toxicity towards a cell line, such as a mammalian, and preferably human, cell line. The results of such studies are often predictive of toxicity in animals, such as mammals, or more specifically, humans. Alternatively, the toxicity of the compounds may be determined in an animal model, such as mice, rats, rabbits, or monkeys. The efficacy of a particular compound may be established using methods, such as in vitro methods, animal models, or human clinical trials.

The present invention further relates to a kit comprising at least one of the compounds according to the present invention, preferably a package or dispenser device with one or more unit dosage forms containing at least one compound according to the present invention, and written instructions for administration or an instruction leaflet. A unit dosage form refers to a product form in which premeasured dosages of the compound are packaged, in contrast to bulk preparations. Preferred unit dosage forms are for example a pill, a tablet, a capsule, a dragee, a gel capsule, a small amount of a liquid formulation, for example in an ampoule, a fast melt formulation, and the like. The package or dispenser device may for example comprise metal or plastic foil, such as a blister pack. The kit may further comprise a notice in a form prescribed by a governmental agency regulating the manufacture, use, or sale of pharmaceutical compositions, which notice is reflective of approval by the agency of the form of the drug for human or veterinary administration. Pharmaceutical compositions comprising a compound of the invention formulated in a compatible pharmaceutical carrier may also be prepared, placed in an appropriate container, and labelled for treatment of an indicated condition.

The technical problem of the present invention is also solved by a method for the preparation of a pharmaceutical composition for the prevention or treatment of a viral infection, in particular an influenza infection, comprising admixing at least one compound according to the present invention with a pharmaceutically acceptable excipient, preferably according to the above.

The technical problem of the present invention is also solved by use of the compounds of the present invention for the preparation of a pharmaceutical composition for the treatment or prevention of a viral infection, in particular an influenza infection, more particularly an influenza infection caused by influenza virus A or B, preferably an influenza virus A.

In a furthermore preferred embodiment the present invention relates to a method of treatment or prevention of a viral infection, in particular an influenza virus infection, wherein an animal or human patient in need thereof is subjected to a preventive administration or treatment with a suitable dose of the compounds according to the present invention for a suitable period of time, in particular with a pharmaceutical composition comprising such a suitable dose of the compounds according to the present invention for a suitable period of time, to prevent or cure the viral infection, in particular the influenza virus infection.

Further preferred embodiments are the subject matter of the subclaims.

The invention is further illustrated by the following non-limiting examples.

### Example 1

To identify novel inhibitors of influenza NA molecular dynamics (MD) simulations and structure-based virtual screening methods were employed.

### METHODS

Molecular Dynamics Simulations: Amber (Case et al., Journal of Computational Chemistry 2005, 26, 1668-1688) was employed for MD simulations using PDB entry 2hty (chain B) (Russell et al., Nature 2006, 443, 45-49) as a template for H5N1 influenza neuraminidase. The protein structure was prepared and protonated according to pH 6.5. Crystal waters were preserved 5 A around the protein. 10 A around the protein a TIP3P water box was placed. Rectangular periodic boundary conditions were defined and electrostatics calculated with the particle mesh Ewald approach (cutoff 10 A). An initial minimization was limited to solvent molecules, applying 500 steepest descent minimization steps and another 500 conjugate gradient cycles. Subsequently, the full system was minimized in 2500 cycles of steepest descent minimization and an equivalent amount of conjugate gradient steps. In the first 50 ps of the simulation, the system was gradually warmed to 300 K, applying weak restraints to the protein. This was followed 10 by a 100 ps equilibration step under constant pressure. The production MD was processed with a 2 fs time step, with SHAKE bond length constraints to all bonds including a hydrogen atom. 20 ns of the trajectory were clustered to identify ten representative protein conformations for ligand docking. The clustering was processed considering the conformational flexibility (i.e., backbone RMSD) of all residues forming the first shell building the active site of NA. In the present teaching, the average linkage algorithm (Shao et al. Journal of Chemical Theory and Computation 2007, 3, 2312-2334) was used for clustering.

Preparation of the Screening Library: The NCI database (approximately 140 000 compounds of the plated version, provided in MDL SD file format including 3D coordinates) was downloaded from the National Cancer Institute website (accessed 24th August 2009). Compounds were ionized at pH 6.5 and the 3D structures were minimized.

Structure-based Virtual Screening: The NCI database was screened for candidate molecules using two X-ray structural models from the PDB (2hu0, a H5N1 holo-NA structure in open conformation and 2hty, an H5N1 apo-NA structure in open conformation; Russell et al., Nature 2006, 443, 45-49) as well as the ten representative frames collected from clustering of the MD trajectory using GOLD (version 4.0, Cambridge Crystallographic Data Centre CCDC, Cambridge, UK; Jones et al., Journal of Molecular Biology 1997, 267, 727-748). Three docking runs per ligand were performed for each of the protein conformations using GoldScore as the scoring function. Only the docking pose obtaining the highest score was preserved for the final hit ranking. Default settings were preserved for all other program parameters.

### RESULTS

Molecular Dynamics Simulations and Virtual Screening: MD simulations were based on PDB 2hty, a structural template of the H5N1 apo-NA in an open X-ray conformation. The setup and processing of the MD simulations has been published elsewhere (Grienke et al., Journal of Medicinal Chemistry 2010, 53, 778-786). The protein backbone RMSD observed for the MD trajectory indicates that, after a short initial phase, the simulated system quickly enters an equilibrium state. 20 ns of the production MD were submitted to clustering using the average linkage algorithm included to the AMBER software package. 95% of all trajectory frames are represented by six clusters (accounting for 27%, 23%, 16%, 14%, 8% and 7% of all clustered frames, respectively). The remaining clusters primarily account for 5 conformational transitions observed during the MD simulations. The approximately 134 000 compounds that passed the data preprocessing workflow were submitted to protein-ligand docking with GOLD. Three docking runs were performed for all compounds on a H5N1 holo-NA, a H5N1 apo-NA X-ray 10 structure and the ten representative MD frames.

The hit lists for all 12 protein conformations were individually inspected, starting from the top-ranked molecule. Overall, 91 unique molecules were identified in total that, from a medicinal chemistry point of view, were found to have reasonable chance of being active in vitro. With the above described approach the compounds listed in table 1 below were identified as candidates for NA inhibitors.

### Example 2

The above identified compounds were analyzed for their bioactivity profile, i. e. their biological activity as NA inhibitors using the following approaches and methods.

### METHODS

Compound Preparations: Oseltamivir carboxylate (GS4071) was kindly provided by F. Hoffmann-La Roche AG (Basel, Switzerland; lot no. RO0640802-002) and zanamivir (GG167) by GlaxoSmithKiine (Uxbridge, UK), which were used as positive controls. Compound stocks were prepared in water and stored at 4 °C. Stock solutions of the compounds listed in table 1 below were prepared in DMSO. All 38 tested small organic molecules were ordered from the National Cancer Institute (NCl) database, USA. Their purity was checked using TLC and LC-MS and revealed to be >95% except for NCl compounds 30456 (90%), 37199 (impure), 66221 (impure), 134132 (about 80%), 134183 (about 85%) and 134134 (impure), which were slightly more impure or a specific percentage could not be determined. This might have influenced their activity in biological testing negatively.

Cells and Viruses: Madin Darby canine kidney (MDCK) cells (Friedrich-Loeffler Institute, Riems, Germany) were grown in Eagle minimum essential medium supplemented with 10% fetal bovine serum, 100 U/ml penicillin, and 100 U/ml streptomycin. Influenza viruses A/Jena/5555/09 and A/Jena/5528/09 were isolated in MDCK cells from nasal swabs obtained from flu patients. Stocks of these clinical H1N1 isolates, of the H1N1 influenza virus A/Puerto Rico/8/34 (A/PR/8/34; Institute of Virology, Philipps University Marburg), and the oseltamivir-resistant human H1N1 isolate A/342/09 (Robert Koch Institute, Berlin, Germany) were propagated in MDCK cells in serum-free medium formulated with 2 µg/ml trypsin and 1.2 mM bicarbonate, aliquoted and stored at -80 °C until use.

Chemiluminescence-based NA Inhibition Assay: NA activity and enzyme inhibition were determined with the commercially available NA-5 Star® kit (Tropix, Applied Biosystems, Darmstadt, Germany) that utilizes a 1,2-dioxetane sialic acid derivative for the substrate, as described in detail recently (Grienke et al., Journal of Medicinal Chemistry 2010, 53, 778-786). To evaluate the concentration required to reduce NA enzyme activity by 50% at least six serial threefold compound dilutions (dilution in NA-Star assay buffer) were tested minimum three times. Oseltamivir and/or zanamivir were included as control compounds on each test plate. At first the enzyme inhibition assay was performed with influenza virus A/PR/8/34. The 50% inhibitory concentration (IC₅₀) values of the compounds according to the present invention are given in table 1, below.

A number of compounds with 50% inhibitory concentration (IC₅₀) lower than 10 µM were tested on two recent H1N1 isolates and/or an oseltamivir-resistant isolate. Also these individual values are given in table 1, below.

Determination of Cytotoxicity: The cytotoxicity of test compounds was determined on 3-day-old MDCK confluent cell monolayers grown in 96-well plates as described previously. Briefly, after removal of cell culture medium, serial twofold dilutions of compounds were added to the cells for 72 h (37°C, 5% CO₂, in triplicate in cell culture medium). Then, the cells were fixed and stained with a crystal violet formalin solution. After dye extraction, the optical density of individual wells was quantified spectrophotometrically at 550/630 nm with a microplate reader. Cell viability of individual compound-treated wells was evaluated as the percentage of the mean value of optical density resulting from six mock-treated cell controls, which was set 100%. The 50% cytotoxic concentration (CC₅₀) was defined as the compound concentration reducing the viability of untreated cell cultures by 50%. The CC₅₀ was calculated from the mean dose-response curve obtained from data of at least two independent experiments. Results are provided in table 1. With the exception of NCI compounds 134137, 134142, and 134144 the CC₅₀ for any measured compound was determined to be above 100 µM.

Biological Testing Results: All compounds identified in this study were investigated using in vitro assays of distinct NA strains to elucidate their bioactivity profile, in particular with regard toward the oseltamivir-resistant strains. The 38 compounds tested exhibit IC₅₀ values below 10 µM for A/PR/8/34; fifteen of them show IC₅₀ values below 0.5 µM for A/PR/8/34. With an IC₅₀ of 40 nM, NCI compound 134142 is the strongest inhibitor of virus strain A/PR/8/34. NCI compounds 65545 and 37200, e.g., show remarkable inhibitory activity against A/PR/8/34 and also against oseltamivir-resistant strains with IC₅₀ values for the oseltamivir-resistant strain A/342/09 of 0.20 µM and 1.45 µM, respectively. The individual IC₅₀ values are given in table 1 below in µM for the different Influenza strains. "X" means value not determined.

Most of the compounds according to the present invention share a naphthalene carboxylic/sulfonic acid ligand core, connected to a central planar system via a diazenyl linker. Interestingly, several distinctive structural features are observable. The linker, e.g., may be exchanged by related structural elements and also by more flexible chains.

The results demonstrate the high potential of protein-ligand docking algorithms used in combination with molecular dynamics simulations. The insight on the conformational behavior of a protein gained from X-ray crystallography is limited and can be extended by molecular dynamics simulations. This allows a more thorough exploration of the accessible conformational space of the protein and its ligand interaction site, which in turn can help to identify novel small organic molecules interacting with the receptor that would not be detectable using exclusively X-ray structural models.

**Table 1**

| Formula | Compound | NCI number | IC₅₀ for A/PR/8/34 | IC₅₀ for A/Jena/5528/09 | IC₅₀ for A/Jena/5555/09 | IC₅₀ for A/342/09 | CC₅₀ (µg/ml) in MDCK cells |
|---|---|---|---|---|---|---|---|
| (I) | 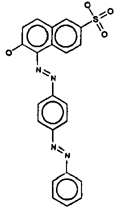 | 65545 | 0.07 | 0.11 | 0.24 | 0.20 | >100 |
| | 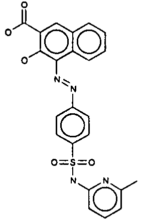 | 134133 | <0.12 | X | X | X | X |
| | 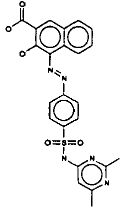 | 134140 | 0.10 | X | X | 0.80 | >100 |
| | 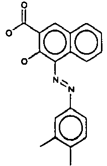 | 134141 | 5.60 | X | X | 20.60 | >100 |
| | 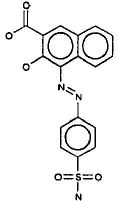 | 134137 | 0.60 | X | X | 1.90 | X |
| | 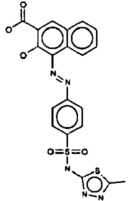 | 134131 | 0.34 | X | X | 0.80 | >100 |
| | 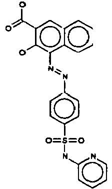 | 134134 | 0.45 | X | X | X | X |
| | 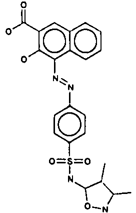 | 134132 | 0.64 | X | X | X | X |
| | 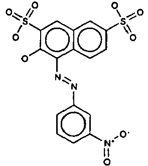 | 45541 | 2.20 | X | X | 7.60 | >100 |
| | 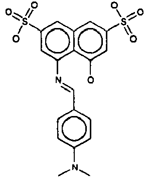 | 37200 | 0.58 | 1.18 | 0.94 | 1.45 | >100 |
| | 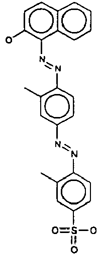 | 66221 | 0.29 | X | X | X | X |
| | 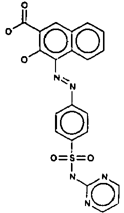 | 134136 | 3.10 | X | X | 14.20 | >100 |
| | 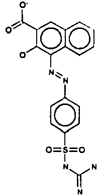 | 134139 | 0.30 | X | X | 7.40 | >100 |
| | 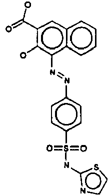 | 134135 | 0.88 | 0.24 | 0.31 | 2.20 | >100 |
| | 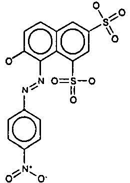 | 45539 | 2.70 | X | X | 9.20 | >100 |
| | 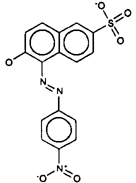 | 45538 | 0.30 | X | X | 4.30 | >100 |
| | 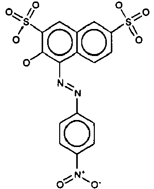 | 45540 | 4.10 | X | X | 9.70 | >100 |
| | 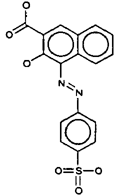 | 134138 | 2.15 | 3.06 | 2.90 | 4.97 | >100 |
| | 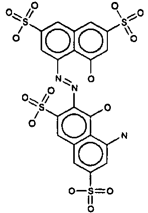 | 162530 | 1.30 | X | X | 5.90 | >100 |
| | 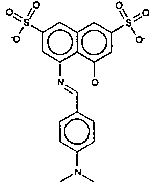 | 37199 | 4.57 | X | X | X | X |
| (II) | 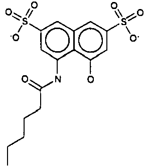 | 97290 | 1.60 | X | X | 4.00 | >100 |
| | 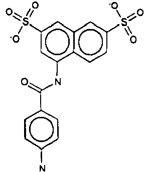 | 37180 | 2.30 | X | X | 9.20 | >100 |
| | 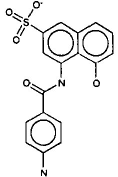 | 37176 | 0.30 | X | X | 1.80 | >100 |
| | 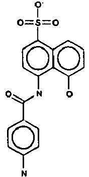 | 37609 | 0.50 | X | X | 1.70 | >100 |
| | 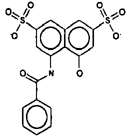 | 16169 | 4.60 | X | X | 7.60 | >100 |
| | 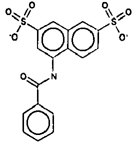 | 37172 | 8.30 | X | X | 8.40 | >100 |
| | 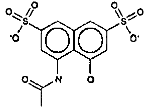 | 74489 | 9.20 | X | X | >24.1 | >100 |
| (III) | 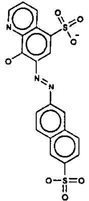 | 87877 | 1.09 | 1.74 | 1.55 | 6.54 | >100 |
| | 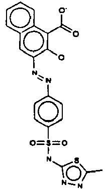 | 134142 | 0.04 | X | X | 1.20 | X |
| | 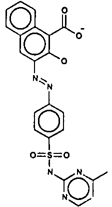 | 134144 | 0.08 | X | X | 3.30 | X |
| | 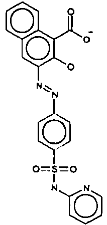 | 134145 | 0.08 | X | X | 3.70 | >100 |
| | 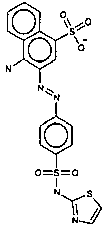 | 31348 | 0.40 | X | X | 3.00 | >100 |
| | 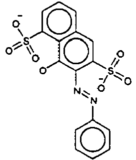 | 45549 | 4.85 | X | X | X | >100 |
| | 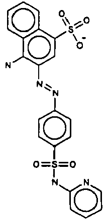 | 30456 | <0.12 | X | X | X | X |
| | 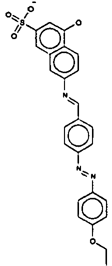 | 37220 | 0.42 | X | X | X | >100 |
| (IV) | 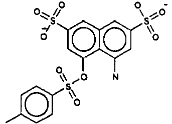 | 16168 | 5.91 | X | X | X | >100 |
| (V) | 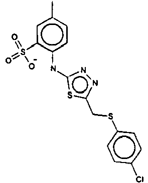 | 369693 | 2.57 | X | X | 3.40 | >100 |
| (VI) | 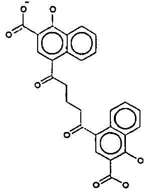 | 292216 | 1.48 | X | X | 6.60 | >100 |

## Claims

1. A compound having a formula selected from the group consisting of 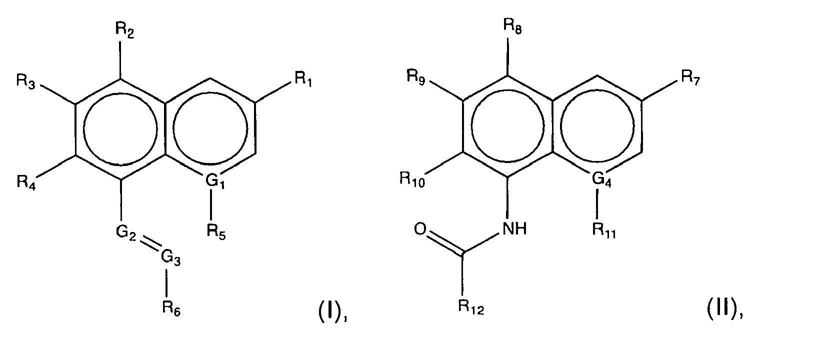 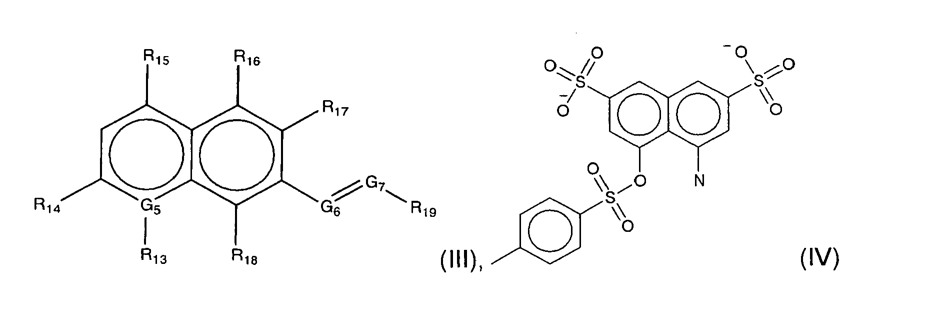 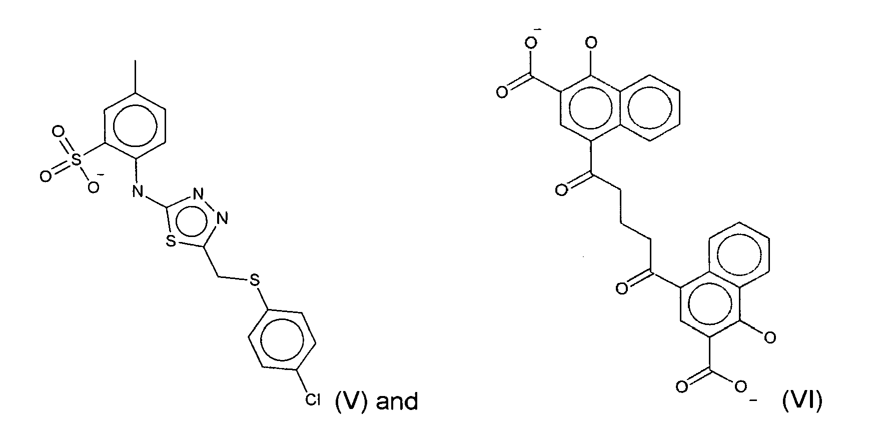 for use in the treatment or prevention of a viral infection, wherein in
(I):
**G1**, **G2** and **G3** are each independently selected from the group consisting of N and C, wherein in case **G1** is N **G1** is not substituted; preferably **G1** is C or substituted C;
**R1**, **R2**, **R3** and **R5** are each independently selected from the group consisting of -SO₃H, -COOH, -OH, -NH₂ and -H;
**R4** is selected from the group consisting of -OH, -NH₂ and -H;
wherein at least one of **R1**, **R2**, **R3** and **R5** is -COOH or -SO₃H or **R4** is -OH or -NH₂; and
**R6** is selected from the group consisting of C1-C6 alkyl chain, phenyl, substituted phenyl, 5-membered or 6-membered heterocycles, substituted 5-membered or 6-membered heterocycles, substituted or non-substituted 5,5 fused bicycles, substituted or non-substituted 6,5 fused bicycles, substituted or non-substituted 6,4 fused bicycles and substituted or non-substituted naphthalenes linked in position 2 or heterocyclic derivatives thereof linked in the corresponding position;
and wherein in (**II**):
**G4** is selected from the group consisting of N and C, wherein in case **G4** is N **G4** is not substituted; preferably **G4** is C or substituted C;
**R7**, **R8**, **R9** and **R11** are each independently selected from the group consisting of -SO₃H, -COOH, -OH, -NH₂ and -H, wherein preferably at least one of **R7**, **R8**, **R9** and **R11** is -COOH or -SO₃H;
**R10** is selected from the group consisting of -OH, -NH₂ and -H; and
**R12** is selected from the group consisting of C1-C6 alkyl chain, phenyl, substituted phenyl, 5-membered or 6-membered heterocycles and substituted 5-membered or 6-membered heterocycles;
and wherein in (**III**):
**G5**, **G6** and **G7** are each independently selected from the group consisting of N and C, wherein in case **G5** is N **G5** is not substituted; preferably **G5** is C or substituted C;
**R13**, **R14** and **R16** are each independently selected from the group consisting of -SO₃H, -COOH and -H,
**R17** is selected from the group consisting of -SO₃H, -COOH, -OH and -H;
wherein preferably at least one of **R13**, **R14**, **R16** and **R17** is -COOH or -SO₃H;
**R15** and **R18** are each independently selected from the group consisting of -OH, -NH₂ and -H; and
**R19** is selected from the group consisting of C1-C6 alkyl chain, phenyl, substituted phenyl, 5-membered or 6-membered heterocycles, substituted 5-membered or 6-membered heterocycles, substituted or non-substituted 5,5 fused bicycles, substituted or non-substituted 6,5 fused bicycles, substituted or non-substituted 6,4 fused bicycles and substituted or non-substituted naphthalenes linked in position 2 or heterocyclic derivatives thereof linked in the corresponding position;
or a pharmaceutically acceptable salt, prodrug or tautomer thereof.

2. The compound of claim 1, wherein the viral infection is an influenza infection.

3. The compound of any one of claims 1 or 2, wherein the influenza virus infection is caused by an influenza virus A or B.

4. The compound of any one of claims 1 to 3, wherein the influenza virus infection is caused by an influenza virus A with a group-1 neuraminidase (N) selected from the group consisting of N1, N4, N5 and N8.

5. The compound according to any one of claims 1 to 4, wherein the influenza virus infection is caused by a H1N1 virus or a H5N1 virus.

6. The compound according to any one of claims 1 to 5, wherein the influenza virus is resistant against oseltamivir.

7. The compound according to any one of claims 1 to 6, wherein the compound has the formula (I) and wherein:
**G1** is C;
**G2** is N;
**R1** is selected from the group consisting of -SO₃H and -H;
**R2** is -H;
**R3** is selected from the group consisting of -H, -COOH and -SO₃H;
**R4** is selected from the group consisting of -H and -OH;
**R5** is selected from the group consisting of -H, -OH and -SO₃H;
wherein at least one of **R1**, **R3** and **R5** is -COOH or -SO₃H; and
**R6** is selected from the group consisting of 4-phenyldiazenylphenyl, 4-(N-(6-methylpyridin-2-yl)sulfamoyl)phenyl, 4-(N-(2,6-dimethylpyrimidin-4-yl)sulfamoyl)phenyl, 4-sulfamoylphenyl, 4-sulfamoylpyridazine, 4-(N-(5-methyl-1,3,4-thiadiazol-2-yl)sulfamoyl)phenyl, 4-(N-(pyridin-2-yl)sulfamoyl)phenyl, 4-(N-(3,4-dimethylisoxazolidin-5-yl)sulfamoyl)phenyl, 3,p-dimethylphenyl, 3-nitrophenyl, 4-nitrophenyl, 4-N,N-dimethylphenyl, 2-methyl-4-((2-methyl-4-sulfophenyl)diazenyl)phenyl, 4-(N-(pyrimidin-2-yl)sulfamoyl)phenyl, 4-(N-carbamimidoylsulfamoyl)phenyl, 4-(N-(thiazol-2-yl)sulfamoyl)phenyl, 4-sulfophenyl and 8-amino-1-hydroxy-3,6-disulfonaphthalen-2-yl.

8. The compound according to claim 7, wherein the compound is selected from the group consisting of the NCI compounds 65545, 134133, 134140, 134137, 134131, 134134, 134132, 134141, 45541, 37200, 66221, 134136, 134139, 134135, 45539, 45538, 45540, 134138, 162530 and 37199.

9. The compound according to claims 7 or 8, wherein the compound is selected from the group consisting of the NCl compounds 65545 and 37200.

10. The compound according to any one of claims 1 to 6, wherein the compound has the formula (**II**) and wherein:
**G4** is C;
**R7**, **R8** and **R9** are each independently selected from the group consisting of -SO₃H and -H;
**R10** is -H;
**R11** is selected from the group consisting of -OH and -H; and
**R12** is selected from the group consisting of pentyl, 4-aminophenyl and phenyl.

11. The compound according to claim 10, wherein the compound is selected from the group consisting of the NCI compounds 97290, 37180, 37176, 37609, 16169, 37172 and 74489.

12. The compound according to any one of claims 1 to 6, wherein the compound has the formula (**III**) and wherein:
**G6** is N;
**R13** is selected from the group consisting of -H and SO₃H;
**R14** is selected from the group consisting of -H and SO₃H;
**R15** is selected from the group consisting of -H and -OH;
**R17** is selected from the group consisting of -H, -OH and SO₃H; and
**R19** is selected from the group consisting of 6-sulfonaphthalen-2-yl, 4-(N-(5-methyl-1,3,4-thiadiazol-2-yl)sulfamoyl)phenyl, 4-(N-(4-methylpyrimidin-2-yl)sulfamoyl)phenyl, 4-(N-(pyridin-2-yl)sulfamoyl)phenyl, 4-(N-(thiazol-2-yl)sulfamoyl)phenyl, phenyl and 4-((4-ethoxyphenyl)diazenyl)phenyl.

13. The compound according to claim 12, wherein the compound is selected from the group consisting of NCl compounds 87877, 134142, 134144, 134145, 31348, 45549, 30456 and 37220.

14. The compound according to any one of claims 1 to 13, wherein the viral infection, preferably influenza infection, is treated by inhibiting the neuraminidase (N) of a virus, in particular an influenza virus.

15. The compound of claim 14, wherein the neuraminidase of the influenza virus is a group-1 neuraminidase (N) selected from the group consisting of N1, N4, N5 and N8.

16. The compound of claims 14 or 15, wherein the neuraminidase of the influenza virus is N1.

17. A pharmaceutical composition comprising at least one of the compounds according to any one of claims 1 to 13 and at least one pharmaceutically acceptable excipient for the treatment or prevention of a viral infection, in particular an influenza infection.

18. A kit comprising at least one of the compounds according to claims 1 to 13 together with written instructions for application or an instruction leaflet or a package.

19. A method for the preparation of a pharmaceutical composition for the treatment of a viral infection, in particular an influenza infection, comprising admixing the compound according to any one of claims 1 to 13 with a pharmaceutically acceptable excipient.
